# FASCICULE DE BREVET EUROPEEN

(11) **EP 1 089 666 B1**
(45) Date de publication et mention de la délivrance du brevet: **11.05.2005**
(21) Numéro de dépôt: 99957140.9
(22) Date de dépôt: 23.06.1999
(51) Int. Cl.: A61B 17/32, A61M 1/00

(54) **APPAREIL DE DISTRIBUTION A DEUX VOIES D'UN LIQUIDE STERILE DE TRAVAIL POUR DES APPLICATIONS MEDICALES**
SPENDERVORRICHTUNG MIT ZWEI ARBEITENDEN PFADEN FÜR STERILE FLÜSSIGKEITEN ZUR MEDIZINISCHEN VERWENDUNG
DISPENSING APPARATUS WITH TWO WORKING STERILE LIQUID PATHS FOR MEDICAL USES

(30) Priorité: 24.06.1998 FR 9808183
(43) Date de publication de la demande: 11.04.2001
(73) Titulaire: Eschmann Holdings Limited, Lancing, West Sussex BN15 8TJ (GB)
(72) Inventeur: GONON, Bertrand, F-69002 Lyon (FR)
(74) Mandataire: Metz, Paul
(86) Numéro de dépôt international: PCT/FR1999/001511
(87) Numéro de publication internationale: WO 1999/066848

(56) Documents cités:
- EP-A- 0 489 496
- EP-A- 0 551 920
- WO-A-94/28807
- WO-A-97/49441
- US-A- 4 655 197
- US-A- 4 676 779
- US-A- 4 702 733
- US-A- 5 322 506
- US-A- 5 605 537

## Description

La présente invention se rapporte à un appareil de distribution sous pression et sous débit contrôlés d'un liquide stérile dans une gamme étendue de pressions et selon des séquences de tirs prédéterminées en régime pulsé ou continu pour au moins une pièce à main en vue d'applications médicales et chirurgicales.

On connaît la technique de l'aquadissection qui utilise un jet haute pression d'un liquide stérile amené à une pièce à main simple ou avec aspiration telle que celle décrite dans la publication de la demande de brevet WO 9703713 au nom de SAPHIR MEDICAL.

Le travail du liquide sous haute pression est un travail de découpe plus ou moins incisive des tissus selon la valeur de la pression.

Il existe d'autres pièces à main qui servent au rinçage ou au lavage, le liquide stérile étant alors distribué sous faible pression selon un débit réglable.

Le liquide stérile est délivré par une source réglable soit en pression soit en débit par exemple celle du générateur décrit dans la publication WO 9428807 au nom de SAPHIR MEDICAL.

Il s'agit d'une enceinte étanche renfermant une poche souple contenant le liquide stérile. Le volume intérieur de l'enceinte est mis sous pression réglable d'un gaz neutre. La force pneumatique de ce gaz expulse le liquide stérile hors de la poche vers le conduit de sortie. Selon le type de pièce à main : dissection ou lavage-rinçage, le liquide sortant est utilisé respectivement en pression ou en débit.

Ce générateur permet de réaliser successivement les deux fonctions correspondant à ces deux catégories d'applications. Le passage de l'une à l'autre nécessite une modification de tubulure et de composant hydraulique.

Ainsi, ce générateur à débit continu est limité à distribuer du liquide stérile pour un seul type de travail à la fois soit de dissection soit de lavage-rinçage.

De plus, le jet est continu et le générateur n'a été prévu que pour alimenter une seule pièce à main à la fois.

Ainsi, il y a lieu de changer le mode de fonctionnement et les conduits à chaque fois que l'on passe d'un régime de pression à un régime de débit c'est-à-dire de l'utilisation d'une pièce à main de dissection à une pièce de lavage-rinçage et inversement.

Le travail s'effectue sous aspiration du liquide de travail par le réseau d'aspiration par exemple celui du bloc opératoire relié à l'évacuation à travers une pompe à vide.

Le générateur étant une source continue, les applications restent limitées à celles d'un jet continu à pression réglable ou à la diffusion en arrosage à des fins de lavage-rinçage ou d'irrigation.

On connaît par le brevet US n° 4,655,197 ATKINSON un générateur de flux pulsatoires de lavage ou d'irrigation délivrés par une pompe à piston et à clapet entraîné par un moteur électrique alternatif en mouvement de va et vient.

Selon cette invention, il existe sur le même appareil une voie séparée d'aspiration dont la dépression est crée par une pompe à membrane entraînée aussi par un moteur électrique alternatif en mouvements de va et vient.

Les deux voies aboutissent sur une même pièce à main permettant d'effectuer des travaux de lavage ou d'irrigation avec aspiration commandée. La fréquence du régime pulsatoire de la pompe de lavage est variable et réglable.

On connaît par ailleurs, par US 4,676,776 l'utilisation d'un dispositif double de pincement pour deux conduits souples permettant d'arrêter par écrasement le passage du flux dans un premier conduit souple dans une première position de ce dispositif et de laisser le deuxième conduit passant et inversement d'arrêter par écrasement le passage du flux dans le deuxième conduit souple dans une deuxième position de ce dispositif et de laisser le premier conduit passant. Ces écrasements résultent de l'appui d'une pièce basculante puis de son homologue sur le conduit souple contre une butée. Ce travail d'écrasement est du type va et vient nécessité par les besoins de débouchage du conduit unique externe.

Par ailleurs, on connaît en chirurgie les avantages des jets pulsés qui permettent d'améliorer la précision du travail de dissection tout en diminuant la pression et la consommation de liquide.

Ce travail résulte de trains successifs d'impulsions périodiques de tir de liquide sous pression, le temps de repos entre les trains successifs étant utilisé pour l'aspiration du liquide projeté et des résidus biologiques.

Si ce régime pulsé permet d'améliorer la précision de la dissection, on contrôle peu la profondeur de l'incision et l'efficacité est mal assurée dans les dissections de faible profondeur. Par ailleurs, la visibilité du champ opératoire peut être notablement améliorée.

L'appareil selon l'invention est défini dans la revendication 1.

La présente invention se rapporte à un appareil fonctionnant avec tout type de sources ou de générateurs de liquide sous pression et avec tout type de moyens d'aspiration. Il est prévu aussi pour fonctionner avec tout type de pièces à main commandées manuellement de différentes façons et notamment celles présentant une commande de l'appareil par discontinuité pneumatique.

L'appareil selon l'invention vise également toutes les applications médicales et chirurgicales dans lesquelles on utilise un jet ou un apport de liquide stérile en vue de réaliser un travail de découpe, de façonnage, de nettoyage, de rinçage, d'irrigation ou tout autre.

On peut citer à titre d'exemple : l'aquadissection, le lavage des tissus, l'irrigation d'une cavité naturelle du corps humain, le façonnage des masses charnelles en chirurgie esthétique, la désagrégation des tumeurs et de nombreuses autres applications.

L'appareil distributeur selon l'invention se veut universel pour les applications médicales et chirurgicales car il peut alimenter des pièces à main de différentes spécificités fonctionnelles dépendant du type de travail demandé. Il peut servir aussi bien dans le cadre d'une opération de chirurgie cardiaque ou du foie ou toute autre opération chirurgicale, que pour la pulvérisation-évacuation d'une tumeur, chacune de ces opérations nécessitant des pièces à main spéciales et différentes et des conditions de fonctionnement et de formes de jet spécifiques.

Par exemple, le travail de dissection et de désagrégation d'une tumeur s'effectue sous aspiration constante destinée à évacuer non seulement le liquide utilisé pour la découpe ou la désintégration, mais aussi les résidus tels que tissus, cellules, matières et liquides organiques.

Pour ce faire, les pièces à main comprennent deux voies, l'une de dissection reliée au liquide stérile sous pression, et l'autre d'évacuation reliée à la source de dépression.

Dans sa version de base, l'appareil de distribution de liquide stérile est relié à une source de liquide sous pression et à des moyens d'aspiration et se compose des moyens généraux suivants :
. une voie de distribution de liquide sous pression reliée à une pièce à main de dissection pour un fonctionnement en régime pulsé-aspiré ou continu ;
. une voie de distribution de liquide sous débit reliée à une pièce à main de lavage-rinçage ou d'irrigation pour un fonctionnement en régime continu à commande manuelle ;
. un module de commande engendrant un régime impulsionnel périodique de fermeture-ouverture des flux sur la voie de pression selon des séquences prédéterminées ;
. des fonctions spécifiques permettant à la pièce à main d'être commandée manuellement par une discontinuité pneumatique.

Plus particulièrement et plus complètement, les principales fonctions de l'appareil peuvent se décrire sommairement de la façon suivante.

Tout d'abord le distributeur comprend au moins deux voies différentes pouvant être reliées à une pièce à main de dissection pour la première et à une pièce de lavage-rinçage pour la deuxième.
A. La voie de dissection comprend :
   . un conduit de distribution du liquide stérile sous pression relié à une pièce à main de dissection ;
   . un conduit de dépression relié à la pièce à main de dissection ;
   . des moyens séquentiels synchronisés de fermeture-ouverture périodique de la dépression agissant sur la voie de dépression ;
   . des moyens synchronisés avec les premiers de mise à l'air périodique de la dépression, agissant sur la voie de dépression ;
   . des moyens synchronisés de fermeture-ouverture périodique du liquide sous pression de dissection.

   Les moyens de commande de fermeture-ouverture périodique peuvent être synchronisés entre eux pour un fonctionnement commun pulsé selon un programme cyclique modifiable.
   Les moyens de commande peuvent être désactivés pour passer à un régime continu.
B. La voie de lavage-rinçage comprend :
   . un conduit de distribution du liquide stérile selon un débit réglable relié à une pièce à main de lavage-rinçage ou d'irrigation ;
   . un conduit de dépression réglable relié à la même pièce à main de lavage-rinçage ;
   . des moyens de réglage du débit du liquide stérile et de la dépression ;
   . des moyens de commande manuelle de fermeture-ouverture du flux de liquide ;
   . des moyens de commande manuelle de fermeture-ouverture de la dépression liés aux moyens de commande précédents pour le liquide.

Cet appareil entièrement nouveau apporte de nombreux avantages dont on indiquera ci-après quelques exemples :
. on dispose de deux voies pouvant fonctionner simultanément indépendamment l'une de l'autre reliée chacune à une pièce à main spécifique dont l'usage peut être simultané ou successif dans le cadre d'un travail sur un même champ opératoire,
. les deux voies peuvent distribuer le fluide de travail et aspirer en régime pulsé ou continu indépendamment l'une de l'autre,
. les séquences programmées produisant les formes d'ondes adaptées des régimes impulsionnels sont préenregistrées et spécifiques d'une application donnée. Elles constituent l'optimum pour cette application,
. les branchements et la disposition des conduits sont facilités,
. la stérilité est garantie à tous les niveaux,
. il est prévu un circuit spécial pour mettre en oeuvre les commandes de fonctionnement des pièces à main à partir de la variation pneumatique d'un fluide,
. les moyens mécaniques assurent les interruptions des flux des fluides dans des conditions stériles,
. il est possible de relier les deux voies à une pièce à main mixte unique,
. l'appareil permet de réaliser un tir par impulsions périodiques de faible durée ou un tir unique d'une seule impulsion,
. le tir périodique de faible durée s'effectue sur un tissu tendu par l'aspiration,
. on contrôle mieux la profondeur de l'incision,
. on bénéficie d'une meilleure visibilité du travail de dissection effectué et du champ opératoire.

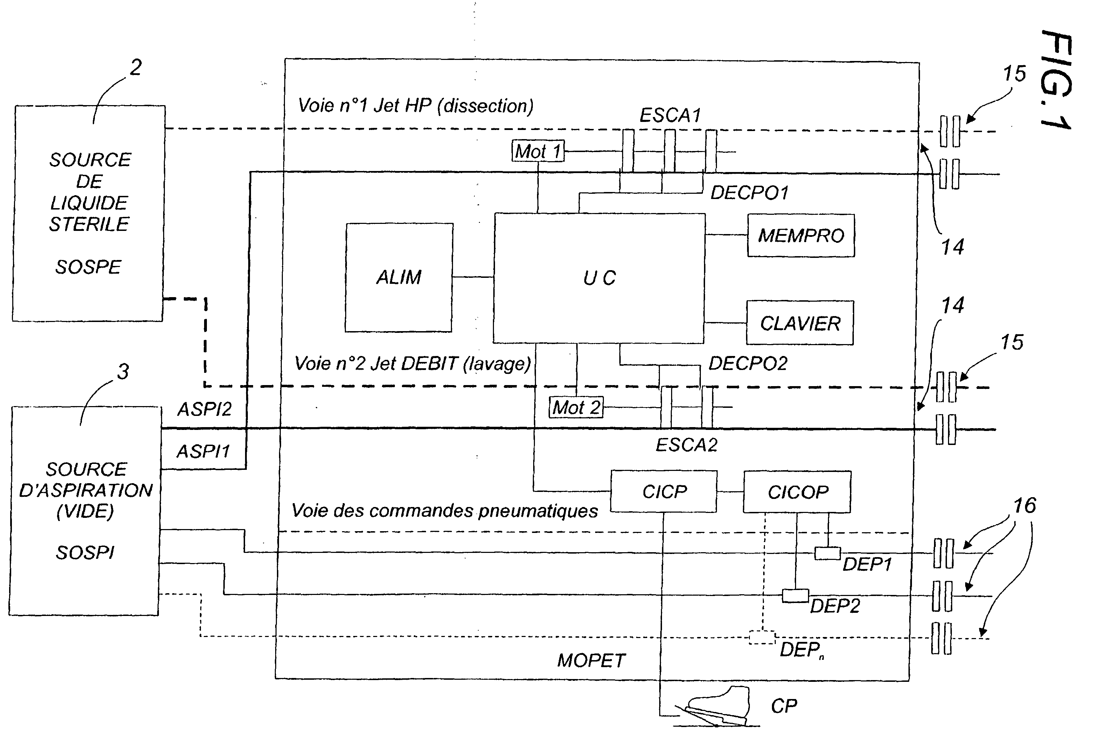

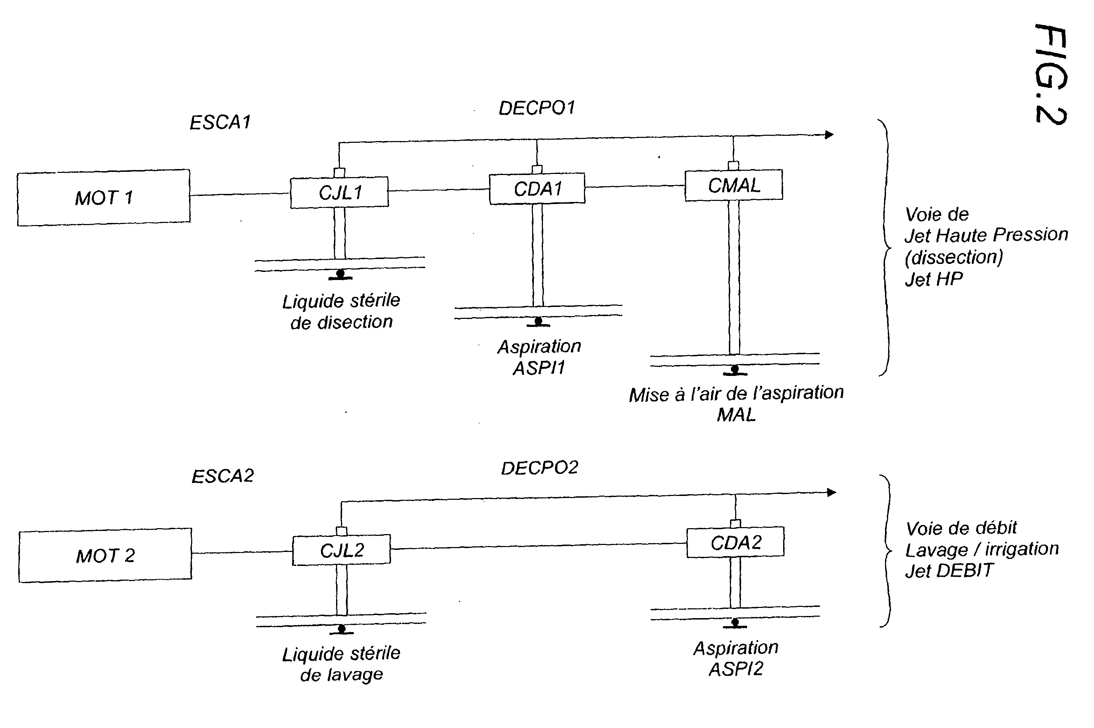

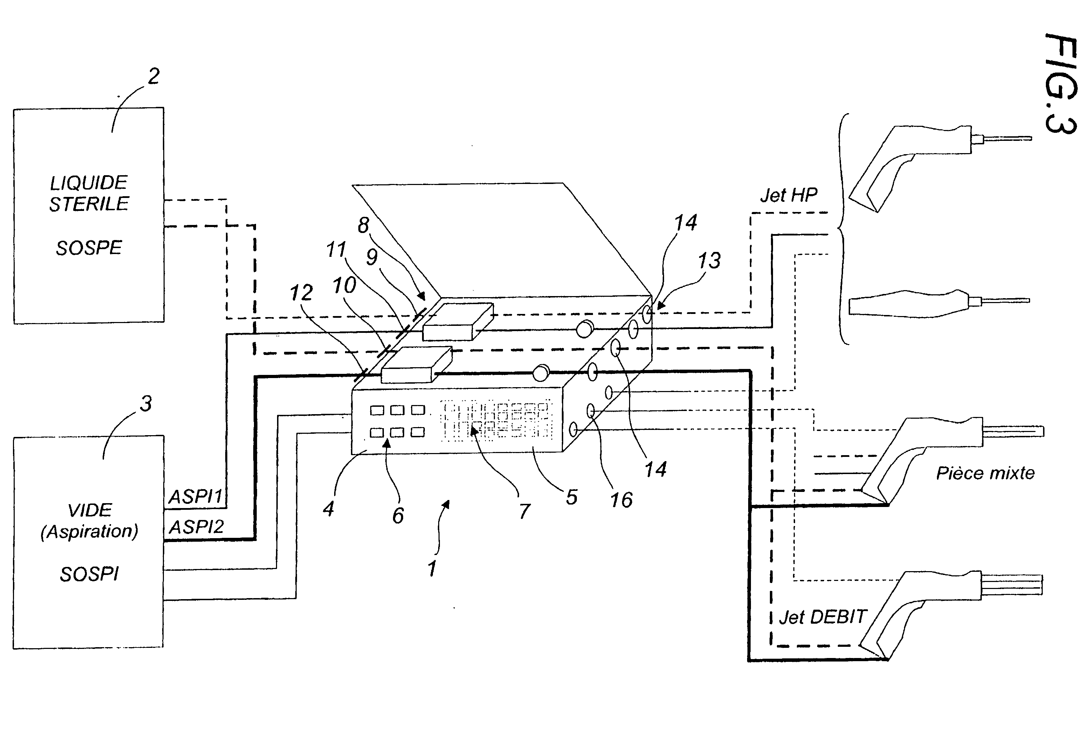

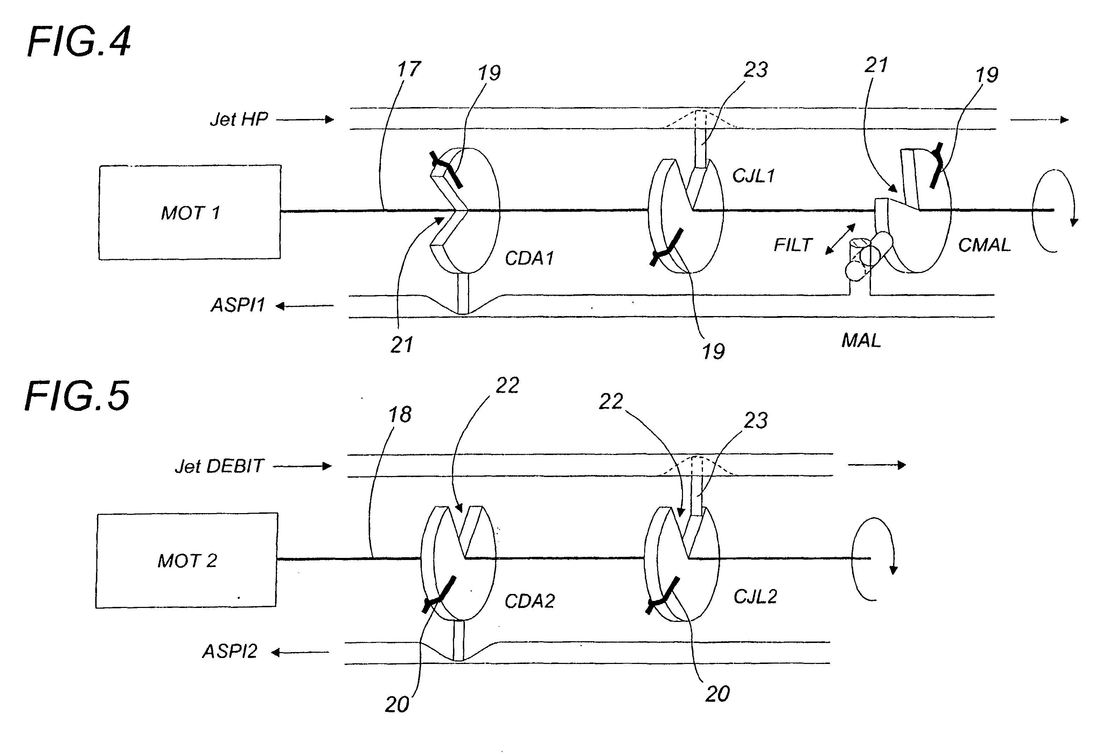

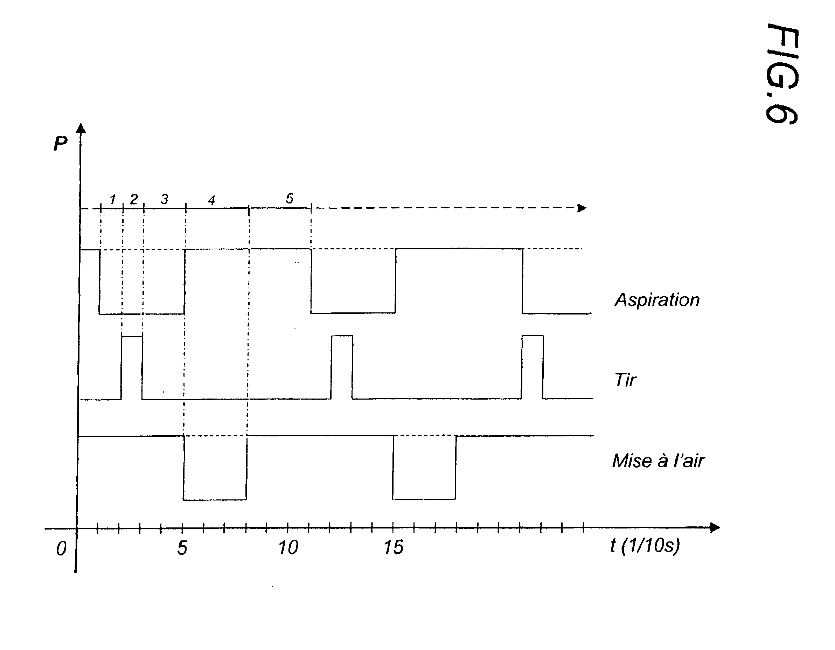

D'autres caractéristiques et avantages de l'invention apparaîtront dans la description qui suit, donnée à titre d'exemple et accompagnée des dessins qui représentent .
. la figure 1 : une vue synoptique générale de l'appareil de distribution selon l'invention ;
. la figure 2 : une vue schématique illustrant le principe sur les deux voies de base de l'appareil selon l'invention ;
. la figure 3 : une vue générale schématique montrant l'appareil dans un montage d'utilisation avec les générateurs et les pièces à main ;
. les figures 4 et 5 : des vues schématiques de détail des deux voies de base avec les ensembles de commande correspondants ;
. la figure 6 : un graphique montrant un exemple de déroulement dans le temps des actions périodiques de commande des trois variables de la voie de dissection.

L'appareil de distribution et d'aspiration selon l'invention se compose d'au moins deux voies de base dont l'une de liquide stérile sous pression notamment de haute pression référencée Jet HP pour des travaux de dissection avec aspiration et l'autre de liquide stérile sous débit référencée jet DEBIT pour des travaux de lavage-rinçage ou d'irrigation avec aspiration. Un ensemble de commande des flux associé à une unité centrale UC et à un bloc mémoire MEMPRO commandent le mode de fonctionnement, le régime et la forme des ondes sur chacune des voies.

L'appareil selon l'invention peut être relié à différentes sources de liquide sous pression telles que des générateurs de nouvelle génération et autres, mais aussi à un simple réservoir disposé en élévation.

De même, l'appareil selon l'invention peut être relié à différentes sources d'aspiration, par exemple les aspirateurs à turbine, les pompes à vide, le réseau de vide des blocs opératoires...

Le liquide employé est stérile. Il peut s'agir par exemple de sérum biologique ou d'une solution de traitement.

La haute pression de travail peut, par exemple, se situer entre 10,et 150 bars.

On a représenté sur le dessin d'une part en traits brisés les liaisons de liquide et d'autre part en traits pleins les liaisons d'aspiration. La différence de grosseur des traits correspond à une différence de débit. Plus le trait est épais plus le débit de cette voie est important.

Comme déjà indiqué, l'appareil 1 selon l'invention peut être relié à deux sources, une source SOSPE de liquide stérile sous pression 2 et une source SOSPI de dépression ou d'aspiration 3. Il se présente sous la forme d'un coffret 4 renfermant tous les circuits de commande et de gestion, les branchements, les ensembles de commande en fermeture-ouverture des flux, ainsi qu'un module spécifique des voies pneumatiques de commande pneumatique des pièces à main qui en sont équipées et au moins une commande à pédale.

Le coffret présente un panneau avant 5 regroupant certaines commandes manuelles 6 et un clavier 7 de communication avec l'unité centrale UC. Sur une des faces latérales 8 du coffret sont regroupées les arrivées de la source de liquide stérile SOSPE selon une entrée de haute pression 9 et une entrée de débit 10 ainsi que les entrées 11 et 12 de la source d'aspiration dans l'appareil. Sur la surface latérale opposée 13 du coffret sont regroupés d'une part les départs 14 des conduits de liaison vers les pièces à main selon une série de raccords tels que 15 et d'autre part les sorties de raccordement 16 des liaisons de commande pneumatique avec les pièces à main qui en sont équipées.

Pour des raisons de sécurité, la face regroupant les départs ou face de sortie peut être condamnée par une porte verrouillable dont la paroi sert de support d'interface pour les raccordements de départ des conduits de liaison avec les pièces à main.

Bien que représenté comme un appareil à deux voies, le nombre de voies de l'appareil de distribution selon l'invention n'est limité que par des considérations physiques et économiques.

De plus, les deux voies de l'appareil peuvent être groupées dans le cas d'une pièce à main mixte comme représenté sur la figure 3 offrant ainsi les fonctions de dissection dans un bloc central et celles de lavage-rinçage ou d'irrigation par une structure coaxiale entourant le bloc central.

L'appareil selon l'invention vise un jet de dissection continu, mais aussi un jet discontinu formé de trains d'impulsions périodiques de tir dont un exemple des formes d'onde est représenté se déroulant dans le temps sur la figure 6 et décrit ci-après ainsi que des séquences à impulsion unique de tir pour des applications particulières.

La figure 1 montre sous la forme d'un schéma synoptique la constitution par ses fonctions principales de l'appareil distributeur selon l'invention.

Une unité centrale UC commande et gère l'ensemble du fonctionnement de l'appareil. Elle est reliée à l'alimentation générale ALIM et à différents périphériques dont un clavier CLAVIER, une mémoire programme MEMPRO, un circuit CICOP d'exploitation des commandes pneumatiques des pièces à main en fonction du programme en service et un circuit CICP d'exploitation d'une commande par une ou plusieurs pédale(s) CP.

L'unité centrale UC commande et gère le fonctionnement des ensembles de commande de chacune des deux voies de base, la voie n° 1 de dissection par jet haute pression référencée jet HP et la voie n° 2 de lavage par écoulement selon un débit réglable référencée jet DEBIT.

Ces ensembles de commande sont des moyens de commande en fermeture-ouverture des flux dans les liaisons fluidiques de l'appareil vers les pièces à main.

Les ensembles de commande dans l'exemple représenté sont deux ensembles électromécaniques ESCA1 et ESCA2 respectivement pour chacune des voies n° 1 et n° 2.

Ces ensembles sont réalisés dans l'exemple représenté comme des dispositifs à cames montées pour un même groupe de cames sur un seul arbre 17 ou 18 entraîné chacun respectivement par un moteur électrique MOT1 et MOT2 propre à chaque voie, alimenté l'un et l'autre à travers l'unité centrale UC à partir de l'alimentation ALIM.

Comme on le verra ci-après, ces moteurs peuvent être par exemple du type à courant continu dits à fréquence variable.

Des détecteurs de position tels que 19 et 20 sont montés pour chaque voie au voisinage des cames. Ils renseignent l'unité centrale UC par les lignes DECP01 et DECP02 sur les positions des cames des deux voies n° 1 et n° 2.

Les cames se présentent par exemple comme celles représentées sous la forme de disques circulaires pleins à secteur angulaire évidé 21 ou 22, d'ouverture angulaire fixe ou réglable. Le chant du disque agit en actionnement sur un moyen de fermeture-ouverture stérile du conduit commandé soit directement soit par l'intermédiaire d'une pièce mobile 23. Pour cette dernière, il peut s'agir d'un levier, d'un doigt, d'une lame, d'une tige ou autre animé d'un mouvement alternatif entre une position de fermeture et une position de libération du conduit. S'agissant de conduits souples pour le transport du fluide stérile dans des conditions stériles ou pour la voie d'aspiration, on utilise de préférence l'obturation par pincement, écrasement ou autre restriction totale par déformation élastique de l'extérieur.

Ainsi, comme représenté, la came peut actionner directement ou indirectement un doigt ou un levier agissant en pincement ou en écrasement sur le conduit souple dans lequel passe le liquide en pression ou en débit ou le fluide gazeux en dépression de la voie d'aspiration pour réaliser les impulsions périodiques du régime impulsionnel.

L'avantage de ce type de commande est la stérilité totale garantie au niveau des interruptions de fermeture-ouverture des conduits.

Pour garantir le maintien du décalage angulaire entre les cames, celles-ci sont montées pour une même voie sur un même arbre pourvu de structures longitudinales par exemple un arbre cannelé.

Le secteur angulaire libre de chaque came correspond à un état inactif de la pièce d'actionnement sur le conduit donc à un passage du fluide. Ce secteur est fixe ou réglable de manière à pouvoir modifier la durée d'action du jet. La fréquence de répétition du phénomène périodique, c'est-à-dire le régime moteur, est donnée par l'unité centrale UC à partir de programmes de fonctionnement préétablis et enregistrés dans MEMPRO correspondant à des valeurs optimales qui se dégagent d'essais représentatifs de cas typiques d'utilisation. On détermine ainsi des rapports cycliques selon les spécialités médicales et chirurgicales et les modes particuliers d'intervention.

On peut modifier ces rapports en changeant le décalage angulaire entre les cames. On peut à cet effet soit les sortir de l'arbre cannelé et les monter autrement décalées angulairement, soit prévoir sur l'arbre des tronçons intermédiaires lisses sur lesquels une ou deux cames peuvent être pivotées librement.

Une des originalités des ensembles de commande en fermeture-ouverture est de pouvoir passer dans un mode de régime continu soit uniquement pour le jet liquide sous pression soit aussi pour l'aspiration moyennant un déplacement angulaire limité de l'arbre commun par exemple cannelé sur lequel sont montées les cames avec ou sans décalage ponctuel de celles-ci entre elles.

Sur l'appareil selon l'invention peuvent se brancher des pièces à main spéciales comportant différents types de commandes manuelles et notamment des commandes pneumatiques basées sur une ou des discontinuité (s) de pression ou de dépression utilisée(s) comme signal de commande pour l'appareil selon l'invention.

Selon ces commandes, des conduits reliés à la source de pression ou de dépression sont utilisés pour initier une modification de pression ou de dépression par une action digitale de l'utilisateur en vue de servir de signal de commande.

Un module annexe MOPET concerne les voies des commandes pneumatiques des pièces à main. Dans ce module passent une pluralité de 1 à n conduits provenant de la source d'aspiration SOSPI destinés à être raccordés en tant que conduits de commande pneumatique aux pièces à main comportant ce type de commande.

Chaque ligne de conduit de commande comporte un capteur ou un détecteur, DEP1, DEP2,...,DEPn, sensible à une variation de pression et/ou de débit et relié par l'intermédiaire du circuit CICOP à l'unité centrale UC.

Les signaux de discontinuité de pression sont interprétés par l'unité centrale UC et exécutés conformément au programme interne.

Pour les capteurs ou détecteurs, il peut s'agir de contacts électriques, magnétiques, résistifs ou autres, de détecteurs et capteurs sensibles à la pression, mais aussi d'éléments sensibles au débit. La modification de ceux-ci ou le signal qu'ils peuvent émettre se traduira par une impulsion de commande.

Le nombre d'impulsions successives peut correspondre à un code de commande ou à une gradation dans l'intensité de la grandeur commandée.

De nombreuses grandeurs peuvent ainsi être commandées relatives au fonctionnement de la pièce à main, par exemple, la pression, le débit, l'aspiration, et le nombre d'impulsions de tir.

Le nombre de conduits de commande pneumatique n'est pas limité. Il correspond aux besoins des pièces à main dont le nombre total détermine la capacité de l'appareil selon l'invention.

On décrira maintenant en détail les ensembles de commande en fermeture-ouverture ESCA1 et ESCA2 des conduits des voies de dissection et de lavage en se référant aux figures 2 à 5 relatives à ces voies.

### Voie n° 1 DISSECTION

L'ensemble de commande de cette voie agit sur un conduit souple haute pression jet HP et sur un conduit souple d'aspiration ASPI1 et sur une mise à l'air MAL à travers un filtre FILT assurant la libération de l'aspiration.

L'ensemble de commande de cette voie se compose d'un moteur MOT1 de courant continu dit à fréquence variable entraînant sur le même arbre, par exemple cannelé, trois cames actionnant des dispositifs de fermeture et de libération d'un conduit de liquide haute pression jet HP et d'aspiration ASPI1.

Les cames sont chacune spécialisées. On distingue une première came de jet liquide haute pression CJL1 qui commande le tir haute pression, une came d'aspiration CDA1 qui commande l'aspiration et une came de mise à l'air CMAL qui commande la mise à l'air périodique MAL sur le conduit d'aspiration ASPI1. Chaque came présente un secteur angulaire plein et un secteur évidé. Le secteur plein commande un dispositif de fermeture du conduit par exemple à poussoir.

L'ensemble de commande ESCA1 de la voie de dissection peut ne comporter que les deux cames CJL1 et CDA1 permettant une poursuite périodique du tir sans relâchement intermédiaire du tissu c'est-à-dire sans mise à l'air périodique MAL.

On peut également commander périodiquement le relâchement du tissu par une neutralisation de la succion provenant de la dépression, d'une autre façon que par la mise à l'air périodique du conduit de dépression.

L'ensemble de commande peut fonctionner en jet continu ou en jet pulsé. Dans le premier cas, il s'agit d'une mise en position fixe des cames dans une configuration donnée. Dans le deuxième cas, il s'agit d'un régime continu de rotation du moteur MOT1 à une vitesse constante donnée provoquant un régime impulsionnel déterminé par la vitesse de rotation du moteur, par les positions respectives des cames décalées angulairement et par l'ouverture angulaire des secteurs évidés des cames.

### Exemple de fonctionnement de la voie de dissection :

### Régime impulsionnel :

A la mise en marche de l'appareil, les cames sont en position ouverte, c'est-à-dire que leur secteur angulaire évidé se trouve en face des conduits, puis le moteur MOT1 est entraîné en rotation à faible vitesse et atteint ensuite la vitesse correspondant à la fréquence du régime sélectionné.

### Régime continu :

L'appareil permet un régime continu sur la voie de dissection. Il fonctionne alors sur commande manuelle par des déplacements angulaires ponctuels et limités des cames entre une position de fermeture et une position d'ouverture du conduit correspondant.

### Voie n° 2 LAVAGE

Cette voie est destinée à toutes les applications nécessitant un débit plutôt qu'une pression. Il s'agit du lavage, mais aussi du rinçage, de l'irrigation des cavités naturelles du corps humain lors d'interventions chirurgicales et de toutes les autres applications de ce type dans les domaines de la chirurgie et de la médecine en général.

L'ensemble de commande de cette voie agit sur un conduit jet DEBIT souple de transport du liquide stérile sous basse pression selon le débit imposé par la pièce à main utilisée et sur un conduit souple d'aspiration ASPI2 relié à la source d'aspiration.

L'ensemble de commande est formé d'un moteur MOT2 à courant continu dit à fréquence variable actionnant l'arbre commun 18, par exemple cannelé, portant deux cames CJL2 et CDA2 solidaires de cet arbre et décalées angulairement entre elles.

Les cames sont disposées, par exemple en opposition de phase, c'est-à-dire décalées de 180° d'angle. Le moteur MOT2 n'est pas utilisé en rotation continue comme le moteur MOT1, mais en commande de déplacement angulaire limité entre une position d'ouverture de l'un des conduits et une position corrélative de lavage avec fermeture de l'aspiration puis d'aspiration avec fermeture du jet de lavage.

Pour ce faire, les cames sont circulaires et présentent un secteur angulaire plein suivi d'un secteur angulaire évidé selon une ouverture angulaire déterminée fixe ou réglable.

Comme précédemment, les cames actionnent en poussée par leur chant un dispositif mécanique d'obturation par exemple un élément poussoir guidé : tige, barre ..., entrant en contact d'écrasement avec le conduit souple en regard en vue d'une fermeture quasi totale puis d'une ouverture lors du passage du secteur évidé.

Afin de pouvoir régler graduellement le débit autrement que par la pièce à main ou par la source de liquide, il peut être prévu une forme à pente curviligne progressive du chant du secteur plein sur la came correspondante.

Des détecteurs de position de la came ou de présence à un endroit donné du secteur plein ou du secteur évidé sont prévus afin de renseigner l'unité centrale sur la position et l'écart ou excursion angulaire à commander. Ces détecteurs sont représentés sur les figures sous la forme d'étriers 20 fixes par rapport au corps de chaque came.

### Exemple de fonctionnement de la voie de lavage :

A la mise en marche de l'appareil les deux cames sont en position de fermeture des conduits.

Une action sur la commande pneumatique d'une pièce à main ou sur la pédale CP provoque la rotation du moteur MOT2 jusqu'à l'ouverture complète du conduit d'aspiration ASPI2 et l'arrêt du moteur lorsque le secteur évidé de la came de dépression CDA2 est situé au niveau du poussoir de commande. Parallèlement, le conduit de liquide jet DEBIT reste fermé.

Une nouvelle action de commande provoquera la rotation limitée du moteur MOT2 jusqu'à la présentation du secteur évidé de la came CJL2 du jet liquide de lavage au niveau du poussoir de commande permettant ainsi à nouveau le passage du liquide stérile de lavage ou de rinçage. Parallèlement, le conduit de dépression se ferme et reste fermé.

Pour améliorer la fiabilité du maintien du décalage angulaire entre les cames on préférera l'utilisation d'un arbre commun cannelé avec éventuellement des tronçons lisses pour permettre la modification du décalage angulaire. Bien entendu, les cames présenteront une ouverture centrale de forme complémentaire permettant de s'ajuster sur les cannelures. Il s'agit d'un arbre cannelé pour chaque groupe de cames ou d'un arbre cannelé unique pour toutes les cames.

A titre de sécurité et de commodité, on peut prévoir sur la face de sortie des conduits une interface de raccordement. Il peut s'agir d'une paroi ou d'un panneau pivotant (non représenté) formant porte de condamnation portant les socles des prises de raccordement elles-mêmes reliées aux extrémités des conduits souples de l'appareil sur lesquels agissent les cames.

Cette paroi permet, lorsqu'elle est rabattue et verrouillée, de couper tout débit dans les conduits d'alimentation et donc une sortie intempestive de liquide alors que la ou les pièces à main ne sont pas encore branchées. La paroi de la porte condamne le passage de tout débit des conduits dans l'appareil au niveau de leurs extrémités avant les raccords de jonction par exemple par pincement ou repliement des conduits. Cette condamnation persiste jusqu'à l'ouverture de la porte.

L'appareil selon l'invention est un appareil multifonctions et multiusages. Cependant, il a été conçu surtout pour mettre en oeuvre sur sa voie n° 1 de haute pression le procédé de génération d'un jet liquide stérile pulsé-aspiré, objet d'une protection parallèle au nom du déposant.

Le procédé, décrit dans cette protection parallèle, se caractérise en ce qu'il met en oeuvre une voie de liquide sous pression et une voie pneumatique d'aspiration et en ce que l'on commande le tir périodique du jet sous pression pendant l'application périodique de l'aspiration.

Le procédé est complété en ce que l'on réalise périodiquement une neutralisation de l'aspiration par vide d'air juste après la fermeture du conduit d'aspiration par exemple par la mise à l'air du conduit d'aspiration et pour des régimes à plus haute fréquence une décharge périodique de la pression résiduelle sur la voie de pression.

Les principales phases caractéristiques de chaque période du train impulsionnel selon de procédé sont les suivantes repérées par les chiffres de 1 à 5 sur la figure 6.
. phase 1 : début de l'aspiration,
. phase 2 : tir du jet sous pression pendant une courte durée à l'intérieur de l'impulsion d'aspiration,
. phase 3 : poursuite de l'aspiration après le tir,
. phase 4 : mise à l'air pendant la coupure de l'aspiration,
. phase 5 : poursuite de la coupure de l'aspiration jusqu'à la prochaine période.

On remarque les caractéristiques générales suivantes à l'observation des figures. Le tir s'effectue avec un certain retard par rapport au début de l'aspiration. Le tir s'effectue de préférence dans la première moitié de la largeur d'impulsion de l'aspiration et s'arrête de préférence avant le début de la deuxième moitié. La mise à l'air a lieu après la coupure de l'aspiration et de préférence mais non obligatoirement juste après cette coupure.

On donne ci-après à titre d'exemple non limitatif les principales valeurs caractéristiques des paramètres du train impulsionnel de jet liquide sous pression pulsé-aspiré correspondant à celui représenté sur la figure 6.
. fréquence de récurrence : 1 Hz
. largeur d'impulsion de tir : 100 ms
. largeur d'impulsion d'aspiration : 400 ms
. repos d'aspiration : 600 ms
. largeur d'impulsion de mise à l'air : 300 ms
. décalage entre la fin de l'impulsion d'aspiration et la mise à l'air : faible.

La mise à l'air est courte et suit immédiatement la fin de l'aspiration.

Les flancs de montée des impulsions ont été représentés verticaux, ce qui est relativement vrai ici dans le cas d'une commande par came et écrasement d'un conduit souple par une tige.

D'autres formes d'ondes différentes sont possibles.

Ainsi, la fréquence de récurrence du régime impulsionnel et la largeur des impulsions peuvent varier. Ces variations dépendent de l'application chirurgicale, à savoir du type d'intervention, d'organe ou de tissu visés ainsi que de la profondeur d'intervention dans le corps humain.

Les commandes correspondant à d'autres formes d'ondes sont préenregistrées dans le module MEMPRO. Il s'agit de formes d'onde caractéristiques d'une application chirurgicale spécifique tant en ce qui concerne l'organe visé que la technique opératoire.

Il doit être noté que plus la largeur de l'impulsion du tir augmente plus celle de l'aspiration doit augmenter pour pouvoir évacuer complètement le liquide et le ou les résidus avant le nouveau tir.

## Revendications

1. Appareil pour la génération de jets pulsés de liquide avec aspiration parallèle sur au moins deux voies à partir d'un liquide stérile de travail, les deux voies pouvant être reliées à au moins une pièce à main d'intervention chirurgicale ou médicale, appareil pouvant être raccordé à une source de liquide stérile sous pression (SOSPE) et à une source de dépression (SOSPI) **caractérisé en ce que** :
. les au moins deux voies de liquide stérile de travail, sont d'une part une voie dite voie sous pression (HP) avec aspiration parallèle pour des travaux de dissection, et d'autre part une voie dite voie sous débit (DEBIT) avec aspiration parallèle pour des travaux de lavage-rinçage ou d'irrigation, chacune de cesdites voies pouvant être raccordée à une pièce à main spécifique, l'une éjectant un ou des jets de liquide sous pression et sortant de l'autre pièce à main un jet en débit, ou les deux voies pouvant être raccordées à une pièce à main mixte de dissection et de lavage-rinçage,
. chacune de cesdites au moins deux voies est constituée de deux conduits, l'un dit conduit d'amenée de liquide et l'autre dit conduit de dépression,
. chacune de cesdites au moins deux voies comporte des moyens de commande pour commander en fermeture-ouverture chacun des conduits desdites voies afin de permettre un fonctionnement de l'appareil en régime périodique à fréquence réglable ou prédéterminée ou en régime continu,
. l'appareil comprend une mémoire (MEMPRO) dans laquelle sont enregistrés des modes de fonctionnement préétablis en liaison avec une unité centrale (UC) pour la génération de jets pulsés ou continus à partir de commandes manuelles (6) pouvant être initiées par l'utilisateur.

2. Appareil générateur selon la revendication 1 **caractérisé en ce que** ladite voie sous pression comporte une commande de mise à l'air périodique de l'aspiration (CMAL) après la coupure de cette aspiration.

3. Appareil générateur selon la revendication 1 **caractérisé en ce que** les moyens de commande de fonctionnement de chacun des conduits de ladite voie sous pression peuvent être synchronisés entre eux pendant l'utilisation pour la génération de jets pulsés-aspirés.

4. Appareil générateur selon la revendication 3 **caractérisé en ce que** ladite voie sous pression comporte une commande de mise à l'air périodique de l'aspiration (CMAL) après la coupure de cette aspiration.

5. Appareil générateur selon l'une quelconque des revendications précédentes **caractérisé en ce que** les moyens de commande des flux (ESCA1, ESCA2) sont électromécaniques.

6. Appareil générateur selon la revendication 5 **caractérisé en ce que** les moyens de commande des flux (ESCA1, ESCA2) sont formés de deux ensembles distincts constitués chacun d'au moins deux cames montées décalées angulairement entre elles sur un arbre commun (17, 18) et actionnées par un moteur électrique (MOT1) pour ladite voie sous pression et par un moteur électrique (MOT2) pour ladite voie sous débit.

7. Appareil générateur selon la revendication 6 **caractérisé en ce que** les cames sont circulaires et présentent au moins un secteur angulaire plein et un secteur angulaire vide ou évidé (21, 22).

8. Appareil générateur selon la revendication 7 **caractérisé en ce que** le secteur évidé (21) ou (22) est d'angle d'ouverture réglable.

9. Appareil générateur selon l'une quelconque des revendications 7 ou 8 **caractérisé en ce que** chaque came est adaptée pour actionner en mouvement par le passage entre le secteur évidé (21, 22) et le secteur plein et inversement un élément mobile (23) qui assure par son déplacement, la fermeture puis l'ouverture du flux dans un conduit de liquide ou dans un conduit de dépression correspondant (ASPI1, ASPI2).

10. Appareil selon l'une quelconque des revendications de 6 à 8 **caractérisé en ce que** l'arbre commun (17, 18) de chaque groupe de cames ou l'arbre commun (17, 18) à toutes les cames est cannelé.

11. Appareil générateur selon l'une quelconque des revendications précédentes **caractérisé en ce que** lesdits conduits d'amenée de liquide desdites voies sous pression et sous débit (ASPI1, ASPI2) sont souples et **en ce que** la fermeture est réalisée par un moyen d'écrasement desdits conduits d'amenée de liquide ou de dépression.

12. Appareil générateur selon l'une quelconque des revendications de 1 à 10 **caractérisé en ce que** lesdits conduits d'amenée de liquide ou de dépression desdites voies sous pression et sous débit (ASPI1) ou (ASPI2) sont souples et **en ce que** la fermeture est réalisée par un moyen de pincement desdits conduits d'amenée de liquide ou de dépression.

13. Appareil générateur selon la revendication 6 **caractérisé en ce que** la voie du liquide sous pression comporte trois cames décalées angulairement entre elles dont une came (CJL1) de fermeture-ouverture du conduit d'amenée de liquide sous pression, une came (CDA1) de fermeture-ouverture du conduit de dépression et une came (CMAL) de mise à l'air périodique de ce conduit de dépression au rythme des jets pulsés pour l'obtention d'un jet pulsé-aspiré suivi d'une détente provoquant le relâchement du tissu.

14. Appareil générateur selon la revendication 13 **caractérisé en ce que** la mise à l'air (MAL) s'effectue à travers un filtre (FILT).

15. Appareil générateur selon la revendication 13 **caractérisé en ce que** ladite voie sous pression ne comprend pas de mise à l'air (MAL).

16. Appareil générateur selon la revendication 6 **caractérisé en ce que** ladite voie sous débit comporte deux cames décalées entre elles dont une came de fermeture-ouverture (CJL2) dudit conduit d'amenée de liquide et une came de fermeture-ouverture (CDA2) dudit conduit de dépression (ASPI2).

17. Appareil générateur selon la revendication 16 **caractérisé en ce que** les cames (CJL2, CDA2) sont portées par le même arbre et décalées angulairement de 180°.

18. Appareil générateur selon l'une quelconque des revendications précédentes de 6 à 10 et de 13 à 17 **caractérisé en ce que** les cames sont équipées d'au moins un détecteur (19, 20) ou d'au moins un capteur de position.

19. Appareil générateur selon la revendication 1 **caractérisé en ce que** les moyens de commande (ESCA1) de ladite voie sous pression sont prévus pour la génération d'un jet continu ou d'un jet pulsé-aspiré périodique à caractéristiques réglables avec mise à l'air périodique (MAL) au rythme de la pulsation.

20. Appareil générateur selon l'une quelconque des revendications précédentes de 5 à 18 **caractérisé en ce que** ladite voie sous débit fonctionnant en continu avec aspiration simultanée ou successive comporte une commande de déplacement angulaire limité des cames.

21. Appareil générateur selon la revendication 1 en combinaison avec au moins une pièce à main pour sa commande manuelle de fonctionnement par l'utilisateur par discontinuité de pression ou de dépression.

22. Appareil générateur selon la revendication 21 **caractérisé en ce que** les commandes manuelles (6) des pièces à main sont des conduits de dépression adaptés pour relier la source de dépression (SOSPI) aux pièces à main en passant par ou à côté d'un capteur ou d'un détecteur correspondant (DEP1, DEP2,..DEPn) sensible à la pression ou à un débit.

23. Appareil générateur selon la revendication 1 en combinaison avec une commande manuelle qui est au moins une pédale (CP).

24. Appareil générateur selon la revendication 1 **caractérisé en ce que** lesdits conduits d'amenée de liquide sortent de l'appareil par une interface de sécurité assurant le raccordement sous la forme d'un panneau pivotant portant les socles des prises de raccordement, ce panneau pivotant formant porte de condamnation permet lorsqu'il est rabattu et verrouillé, d'arrêter tout passage dans les conduits d'amenée de liquide pendant l'utilisation de l'appareil.

25. Appareil générateur selon la revendication 24 **caractérisé en ce que** le passage de fluide dans lesdits conduits d'amenée de liquide dans lesdites voies sous pression et sous débit est arrêté par pincement ou repliement de ces conduits suite au rabattement par pivotement du panneau pivotant formant porte de condamnation.

## Patentansprüche

1. Vorrichtung zum Erzeugen von gepulsten Flüssigkeitsstrahlen mit paralleler Ansaugung über wenigstens zwei Pfade, welche von einer sterilen Arbeitsflüssigkeit ausgehen, wobei die beiden Pfade mit wenigstens einem Handstück zum chirurgischen oder medizinischen Einsatz verbunden sein können, wobei die Vorrichtung an eine unter Druck stehende, sterile Flüssigkeitsquelle (SOSPE) und an eine Unterdruckquelle (SOSPI) angeschlossen sein kann, **dadurch gekennzeichnet, daß**
- die wenigstens zwei sterilen Arbeitsflüssigkeitspfade, einerseits ein sogenannter unter Druck stehender Pfad (HP) mit paralleler Ansaugung für Sezierungsarbeiten, andererseits ein sogenannter Auslaßpfad (DEBIT) mit paralleler Ansaugung für Wasch-/Spülarbeiten oder Berieselungen sind , wobei jeder der Pfade an ein spezielles Handstück angeschlossen sein kann, wobei das eine einen oder mehrere Flüssigkeitsstrahlen unter Druck ausstößt und aus dem anderen Handstück einen Auslaßstrahl ausgibt, oder die beiden Pfade an ein kombiniertes Handstück zum Sezieren und waschen/Spülen angeschlossen sein können ;
- jeder der wenigstens zwei Pfade von zwei Leitungen gebildet ist, wobei die eine Leitung Flüssigkeit zuführt und die andere Leitung unter Unterdruck steht;
- jeder der wenigstens zwei Pfade mit Steuermitteln zum Zu- und Aufsteuern einer jeden Leitung der Pfade ausgestattet ist, um einen periodischen Betrieb mit einstellbarer oder vorherbestimmbarer Frequenz oder einen fortwährenden Betrieb der Vorrichtung zu ermöglichen;
- die Vorrichtung einen Speicher (MEMPRO) aufweist, in welchem vorab festgelegte Betriebsarten abgespeichert sind, welcher mit einer Zentraleinheit (UC) zum Erzeugen von gepulsten oder fortwährenden Strahlen verbunden ist, ausgehend von manuellen Steuerungen (6), welche von dem Benutzer eingegeben werden können.

2. Erzeugervorrichtung nach Anspruch 1, **dadurch gekennzeichnet, daß** der unter Druck stehende Pfad eine Steuerung zur periodischen Belüftung der Ansaugung (CMAL) nach der Abtrennung dieser Ansaugung aufweist.

3. Erzeugervorrichtung nach Anspruch 1, **dadurch gekennzeichnet, daß** die Betriebssteuermittel einer jeden Leitung des unter Druck stehenden Pfades zur Erzeugung von gepulsten angesaugten Strahlen während des Betriebs miteinander synchronisiert sein können.

4. Erzeugervorrichtung nach Anspruch 3, **dadurch gekennzeichnet, daß** der unter Druck stehende Pfad eine Steuerung zur periodischen Belüftung der Ansaugung (CMAL) nach der Abtrennung dieser Ansaugung aufweist.

5. Erzeugervorrichtung nach einem der vorangehenden Ansprüche, **dadurch gekennzeichnet, daß** die Flußsteuermittel (ESCA1, ESCA2) elektromechanisch sind.

6. Erzeugervorrichtung nach Anspruch 5, **dadurch gekennzeichnet, daß** die Flußsteuermittel (ESCA1, ESCA2) von zwei getrennten Einheiten gebildet sind, welche jeweils von wenigstens zwei unter gegenseitigem Winkelversatz auf einer gemeinsamen Welle (17, 18) angeordneten Kurvenscheiben gebildet sind, welche von einem Elektromotor (MOT1) für den unter Druck stehenden Pfad und von einem Elektromotor (MOT2) für den Auslaßpfad betätigt sind.

7. Erzeugervorrichtung nach Anspruch 6, **dadurch gekennzeichnet, daß** die Kurvenscheiben kreisförmig ausgebildet sind und wenigstens einen ausgefüllten Winkelbereich sowie einen freien oder ausgesparten Winkelbereich (21, 22) aufweisen.

8. Erzeugervorrichtung nach Anspruch 7, **dadurch gekennzeichnet, daß** der ausgesparte Bereich (21, 22) einen steuerbaren Öffnungswinkel aufweist.

9. Erzeugervorrichtung nach einem der Ansprüche 7 oder 8, **dadurch gekennzeichnet, daß** jede Kurvenscheibe mittels des Durchgangs durch den ausgesparten Bereich (21, 22) und den ausgefüllten Bereich und umgekehrt zum Bewegungsantrieb eines beweglichen Elementes (23) ausgebildet ist, welches infolge seiner Verlagerung ein Schließen und sodann ein Öffnen des Flusses in einer Flüssigkeitsleitung oder in einer entsprechenden Unterdruckleitung (ASPI1, ASPI2) sicherstellt.

10. Vorrichtung nach einem der Ansprüche 6 bis 8, **dadurch gekennzeichnet, daß** die gemeinsame Welle (17, 18) einer jeden Gruppe von Kurvenscheiben oder die gemeinsame Welle (17, 18) sämtlicher Kurvenscheiben gerillt ist.

11. Erzeugervorrichtung nach einem der vorangehenden Ansprüche, **dadurch gekennzeichnet, daß** die Leitungen zum Zuführen von Flüssigkeit der unter Druck stehenden Pfade und der Auslaßpfade (ASPI1, ASPI2) nachgiebig sind, und daß das Schließen mittels eines Quetschmittels der Leitungen zum Zuführen von Flüssigkeit oder der Unterdruckleitungen durchführbar ist.

12. Erzeugervorrichtung nach einem der Ansprüche 1 bis 10, **dadurch gekennzeichnet, daß** die Leitungen zum Zuführen von Flüssigkeit oder die Unterdruckleitungen der unter Druck stehenden Pfade und der Auslaßpfade (ASPI1, ASPI2) nachgiebig sind, und daß das Schließen mittels eines Klemmittels der Leitungen zum Zuführen von Flüssigkeit oder der Unterdruckleitungen durchführbar ist.

13. Erzeugervorrichtung nach Anspruch 6, **dadurch gekennzeichnet, daß** der unter Druck stehende Flüssigkeitspfad drei unter gegenseitigem Winkelversatz angeordnete Kurvenscheiben aufweist, von welchen eine Kurvenscheibe (CJL1) zum Schließen/Öffnen der unter Druck stehenden Leitung zum Zuführen von Flüssigkeit, eine Kurvenscheibe (CDA1) zum Schließen/Öffnen der Unterdruckleitung und eine Kurvenscheibe (CMAL) zum periodischen Belüften dieser Unterdruckleitung im Rhythmus der gepulsten Strahlen dient, um einen gepulsten angesaugten Strahl zu erhalten, welcher von einem Druckgefälle gefolgt ist, welcher eine Lockerung des Gewebes verursacht.

14. Erzeugervorrichtung nach Anspruch 13, **dadurch gekennzeichnet, daß** die Belüftung (MAL) durch ein Filter (FILT) hindurch geschieht.

15. Erzeugervorrichtung nach Anspruch 13, **dadurch gekennzeichnet, daß** der unter Druck stehende Pfad keine Belüftung (MAL) aufweist.

16. Erzeugervorrichtung nach Anspruch 6, **dadurch gekennzeichnet, daß** der Auslaßpfad zwei unter gegenseitigem Versatz angeordnete Kurvenscheiben aufweist, von welchen eine Kurvenscheibe (CJL2) zum Schließen/Öffnen der Leitung zum Zuführen von Flüssigkeit und eine Kurvenscheibe (CDA2) zum Schließen/Öffnen der Unterdruckleitung (ASPI2) dient.

17. Erzeugervorrichtung nach Anspruch 16, **dadurch gekennzeichnet, daß** die Kurvenscheiben (CJL2, CDA2) auf derselben Welle sitzen und einen Winkelversatz von 180° aufweisen.

18. Erzeugervorrichtung nach einem der vorangehenden Ansprüche 6 bis 10 und 13 bis 17, **dadurch gekennzeichnet, daß** die Kurvenscheiben mit wenigstens einem Detektor (19, 20) oder mit wenigstens einem Wegfühler ausgestattet sind.

19. Erzeugervorrichtung nach Anspruch 1, **dadurch gekennzeichnet, daß** die Steuermittel (ESCA1) des unter Druck stehenden Pfades zum Erzeugen eines fortwährenden Strahls oder eines gepulsten angesaugten periodischen Strahls von einstellbarer Funktion mit der periodischen Belüftung (MAL) im Rhythmus der Pulsation vorgesehen sind.

20. Erzeugervorrichtung nach einem der vorangehenden Ansprüche 5 bis 18, **dadurch gekennzeichnet, daß** der fortwährend mit simultaner oder sukzessiver Ansaugung arbeitende Auslaßpfad eine Steuerung zum begrenzten Winkelversatz der Kurvenscheiben aufweist.

21. Erzeugervorrichtung nach Anspruch 1 in Kombination mit wenigstens einem Handstück zur manuellen Betriebssteuerung durch den Benutzer durch Diskontinuitäten des Druckes oder des Unterdruckes.

22. Erzeugervorrichtung nach Anspruch 21, **dadurch gekennzeichnet, daß** die manuellen Steuerungen (6) der Handstücke Unterdruckleitungen sind, welche zur Verbindung der Unterdruckquelle (SOSPI) mit den Handstücken unter Durchgang durch einen oder vorbei an einem entsprechenden druckempfindlichen Fühler oder Detektor (DEP1, DEP2, ..DEPn) oder mit einem Auslaß ausgebildet sind.

23. Erzeugervorrichtung nach Anspruch 1 in Kombination mit einer manuellen Steuerung, welche von wenigstens einem Pedal (CP) gebildet ist.

24. Erzeugervorrichtung nach Anspruch 1, **dadurch gekennzeichnet, daß** die Leitungen zum Zuführen von Flüssigkeit über eine Sicherheitsverbindungsstelle von der Vorrichtung ausgehen, die einen Anschluß in Form einer schwenkbaren Platte sicherstellt, welche die Sockel der Anschlußstutzen trägt, wobei es die eine Schließpforte bildende, schwenkbare Platte ermöglicht, jeglichen Durchlaß durch die Leitungen zum Zuführen von Flüssigkeit während des Betriebs der Vorrichtung zu unterbrechen, wenn sie umgeklappt und verriegelt ist.

25. Erzeugervorrichtung nach Anspruch 24, **dadurch gekennzeichnet, daß** der Fluiddurchlaß durch die Leitungen zum Zuführen von Flüssigkeit in den unter Druck stehenden Pfaden und den Auslaßpfaden durch Abklemmen oder Umbiegen dieser Leitungen infolge Umklappen durch Verschwenken der eine Schließpforte bildenden, schwenkbaren Platte unterbrochen ist.

## Claims

1. Apparatus for the generation of pulsated liquid jets with parallel aspiration on at least two ways based on a working sterile liquid, both ways may be connected to at least one hand tool for surgical or medical operation, this apparatus may be connected to a source of pressurized sterile liquid (SOSPE) and to a source of depression (SOSPI) **characterized in that**:
. the at least two ways for working sterile liquid, are, on the one hand, a pressurized way (HP) with parallel aspiration for dissection, and, on the other hand, a way under flow (FLOW) with parallel aspiration for washing-rinsing or irrigation, either way may be connected to a specific hand tool, one ejecting one or more fluid flushes under pressure and with a jet flow emitted out of the other hand tool, or the two ways may be connected to a composite hand tool for dissection and washing-rinsing,
. each one of the at least two ways includes two conduits, one called the liquid feeding conduit and the other called the depression conduit,
. each one of the at least two ways comprises a control panel to operate opening and closing of each conduit of the ways in order to allow for operation of the apparatus with an adjustable or predetermined frequency rate or at a continuous rate,
. the apparatus includes a memory (MEMPRO) in which preset operating modes are recorded in connection with a central processing unit (CPU) for the generation of pulsated or continual jets from manual controls (6) which can be initiated by the user.

2. Generating apparatus according to claim 1 **characterized in that** the pressurized way includes a control for periodical venting of aspiration (CMAL) after the cutting off of this aspiration.

3. Generating apparatus according to claim 1 **characterized in that** the operating controls for each conduit of the pressurized way may be synchronized between them during the generation of pulsated-aspired jets.

4. Generating apparatus according to claim 3 **characterized in that** the pressurized way includes a control for periodical venting of aspiration (CMAL) after the cutting off of this aspiration.

5. Generating apparatus according to any of the preceding claims, **characterized in that** the flow controls (ESCA1 and ESCA2) are electromechanical.

6. Generating apparatus according to claim 5 **characterized in that** the flow controls (ESCA1 and ESCA2) include two distinct sets each with at least two cams assembled at an angle on a single shaft (17 or 18) and driven by an electric motor (MOT1) for the pressurized way and by an electric motor (MOT2) for the way under flow.

7. Generating apparatus according to claim 6 **characterized in that** the cams are circular and present at least one full angular sector and an empty or hollow angular sector (21 or 22).

8. Generating apparatus according to claim 7 **characterized in that** the hollow sector (21 or 22) has an adjustable aperture.

9. Generating apparatus according to claims 7 or 8 **characterized in that** each cam is adapted to actuate moving by the passage between the hollow sector (21 or 22) and the full sector and conversely a variable component (23), the movement of the latter closes and opens flow in a conduit of liquid or a corresponding depression conduit (ASPI1 or ASPI2).

10. Apparatus according to any of the claims from 6 to 8 **characterized in that** the common shaft (17) either (18) for each group of cams or (17 or 18) for all cams, is grooved.

11. Generating apparatus according to any of the preceding claims **characterized in that** the pressurized or flow liquid conduits or the depression conduits (ASPI1 or ASPI2) are flexible and **in that** closing is carried out by crushing of the liquid or depression conduits.

12. Generating apparatus according to any of the claims from 1 to 10 **characterized in that** the pressurized or flow conduits or the depression conduits (ASPI1 or ASPI2) are flexible and **in that** closing is carried out by pinching of the liquid or depression conduits.

13. Generating apparatus according to claim 6 **characterized in that** the pressurized liquid way includes three cams placed at angles, including one cam (CJL1) to open and close the pressurized liquid conduit, one cam (CDA1) to open and close the depression conduit, and one cam (CMAL) for periodical venting of the depression conduit at the rate of the jets pulsated to obtain a pulsated-aspired jet followed by a pause leading to relaxation of the material.

14. Generating apparatus according to claim 13 **characterized in that** the venting (MAL) is carried out through a filter (FILT).

15. Generating apparatus according to claim 13 **characterized in that** the pressurized way or the dissection way does not include venting (MAL).

16. Generating apparatus according to claim 6 **characterized in that** the under flow way includes two split cams of which one is a cam to open and close (CJL2) the liquid conduit and the other is a cam to open and close (CDA2) the depression conduit (ASPI2).

17. Generating apparatus according to claim 16 **characterized in that** cams (CJL2 and CDA2) are at 180° on the same shaft.

18. Generating apparatus according to any of the preceding claims from 6 to 10 and 13 to 17 **characterized in that** the cams are equipped with at least one detector (19 or 20) or at least a position encoder.

19. Generating apparatus according to claim 1 **characterized in that** the controls (ESCA1) of the pressurized way are provided for the generation of a continuous jet or a periodic pulsated-aspired jet with adjustable characteristics and periodical venting (MAL) at the rate of the pulsation.

20. Generating apparatus according to any of the preceding claims from 5 to 18 **characterized in that** the under flow way functioning uninterrupted with simultaneous or successive aspiration includes a control for limited angled movement of the cams.

21. Generating apparatus according to claim 1 in combination with at least one hand tool for manual control of operation by the user via interrupted pressure or depression.

22. Generating apparatus according to claim 21 **characterized in that** the manual controls (6) of hand tools are depression conduits adapted to connect the source of depression (SOSPI) to hand tools while passing by or beside a sensor or a corresponding detector (DEP1, DEP2..DEPn) for pressure or flow.

23. Generating apparatus according to claim 1 in combination with a manual control which is at least a pedal (CP).

24. Generating apparatus according to claim 1 **characterized in that** the liquid conduits leave the apparatus via a safety interface ensuring connection with a pivoting panel supporting the bases of the connection terminals. This pivoting panel forms a locking gate stopping any liquid from entering the conduits during use of the apparatus when folded back and locked.

25. Generating apparatus according to the claim 24 **characterized in that** the passage of fluid in the liquid conduits in the pressurized or under flow ways is stopped by pinching or folding of these conduits following the folding back by pivoting of the pivoting panel forming a locking gate.
